# NEUE EUROPÄISCHE PATENTSCHRIFT

(11) **EP 2 464 394 B2**
(45) Veröffentlichungstag und Bekanntmachung des Hinweises auf die Entscheidung über den Einspruch: **17.10.2018**
(45) Hinweis auf die Patenterteilung: 09.09.2015
(21) Anmeldenummer: 10740147.3
(22) Anmeldetag: 02.07.2010
(51) Int. Cl.: A61M 1/00

(54) **AM KÖRPER EINES BENUTZERS TRAGBARE VORRICHTUNG ZUR BEREITSTELLUNG VON UNTERDRUCK FÜR MEDIZINISCHE ANWENDUNGEN**
DEVICE THAT CAN BE WORN ON THE BODY OF A USER AND THAT PROVIDES VACUUM FOR MEDICAL USES
SYSTÈME POUVANT ÊTRE PORTÉ SUR LE CORPS D'UN UTILISATEUR ET SERVANT À PRODUIRE UNE DÉPRESSION POUR DES APPLICATIONS MÉDICALES

(30) Priorität: 12.08.2009 DE 102009038130
(43) Veröffentlichungstag der Anmeldung: 20.06.2012
(73) Patentinhaber: Paul Hartmann Aktiengesellschaft, 89522 Heidenheim (DE); ATMOS MedizinTechnik GmbH & Co. KG, 79853 Lenzkirch (DE)
(72) Erfinder: ECKSTEIN, Axel, 89522 Heidenheim (DE); HOFSTETTER, Jürgen, 89522 Heidenheim (DE); MÖLLER, Mario, 79100 Freiburg (DE); HEER, Andreas, 79780 Stühlingen-Grimmelshofen (DE); WEGNER, Simon, 78183 Mundelfingen (DE)
(74) Vertreter: DREISS Patentanwälte PartG mbB
(86) Internationale Anmeldenummer: PCT/EP2010/004011
(87) Internationale Veröffentlichungsnummer: WO 2011/018133

(56) Entgegenhaltungen:
- EP-A1- 1 905 465
- WO-A1-80/02182
- WO-A1-97/18007
- WO-A1-2007/013064
- WO-A1-2007/087808
- WO-A1-2009/004367
- WO-A1-2009/004368
- WO-A1-2009/004369
- WO-A1-2009/004370
- WO-A1-2009/004371
- WO-A1-2009/004371
- WO-A1-2009/071924
- WO-A2-2009/004289
- WO-A2-2009/021047
- US-A- 5 496 299
- US-A1- 2002 198 503
- US-A1- 2008 004 549
- US-A1- 2009 079 701
- US-A1- 2009 157 019
- US-A1- 20080 004 549
- US-A1- 20080 082 059
- US-A1- 20090 079 701
- US-A1- 20090 157 019

## Beschreibung

Die Erfindung betrifft eine am Körper eines Benutzers tragbare Vorrichtung zur Bereitstellung von Unterdruck für medizinische Anwendungen, insbesondere zur Unterdruckbehandlung von Wunden am menschlichen oder tierischen Körper, mit einer Unterdruck erzeugenden Einrichtung und einem nach Gebrauch wegwerfbaren Einweg-Behälter zur Aufnahme von Körperflüssigkeiten, insbesondere von aus einer Wunde abgesaugten Wundsekreten, und mit einem Anschluss für eine hierfür zum Körper führende Saugleitung, wobei die Unterdruck erzeugende Einrichtung in oder an einem ersten Gehäuseteil der Vorrichtung angeordnet ist und der Behälter einen zweiten Gehäuseteil der Vorrichtung bildet, und die Gehäuseteile lösbar gegeneinander fixierbar sind, und wobei die Vorrichtung Befestigungsmittel aufweist, so dass sie am Körper des Benutzers tragbar und mitführbar ist, wobei beide Gehäuseteile (4, 8) im wesentlichen scheibenförmig ausgebildet sind und über eine im wesentlichen vertikal orientierte Trennebene (14) gegeneinander anliegen.

Vorrichtungen zur Unterdruckwundbehandlung sind bereits mehrfach bekannt geworden, insbesondere durch US 2004/0073151 A1, WO 2009/047524 A2, WO 2007/030599 A2 oder EP 1 905 465 A1. Eine gattungsgemäße Vorrichtung ist bekannt aus US 5,496,299; diese Vorrichtung dient aber der Sammlung und Reinfusion von aus einer postoperativen Wunde abgesaugtem Blut bei demselben Patienten.

Bei Vorrichtungen zur Unterdruckbehandlung von Wunden kommuniziert eine Unterdruck erzeugende Einrichtung über eine Saugleitung mit der Wunde oder der Wundumgebung, wobei ein luftundurchlässiges Abdeckmaterial zum luftdichten Verschließen der Wunde und der Wundumgebung vorgesehen ist, so dass ein Unterdruck im Wundraum herstellbar und Flüssigkeiten aus dem Wundraum in den genannten Behälter absaugbar sind.

Der Begriff eines Unterdrucks bezeichnet im Zusammenhang mit der vorliegenden Erfindung einen gegenüber dem Umgebungsluftdruck (atmosphärischer Luftdruck) erniedrigten Luftdruck insbesondere innerhalb eines Wundverbands. Das Abdeckmaterial eines Wundverbands zum luftdichten Verschließen eines Wundraums muss daher so ausgebildet sein, dass es der sich einstellenden Druckdifferenz standzuhalten vermag, damit der Unterdruck im Wundraum überhaupt angelegt und aufrechterhalten werden kann. Im Bereich der Unterdrucktherapie in der Wundbehandlung wird der Unterdruck quantitativ als Druckdifferenz zwischen dem Umgebungsluftdruck und dem unterhalb des Abdeckmaterials angelegten Luftdruck angegeben. Typischerweise beträgt diese Druckdifferenz im Bereich der Unterdrucktherapie höchstens 250 mm Hg (mm Quecksilbersäule) (1 mm Hg = 1 Torr. = 133,322 Pa). Dieser Unterdruckbereich bis höchstens 250 mm Hg hat sich als für die Wundheilung geeignet erwiesen. Ein bevorzugter Unterdruckbereich liegt zwischen 10 und 150 mm Hg.

Der unter Verwendung der Vorrichtung an die Wunde angelegte Unterdruck kann bei einer typischen Unterdruckbehandlung entweder zeitlich im Wesentlichen konstant gehalten werden, oder er kann zeitlich verändert, insbesondere zyklisch verändert werden, was über eine entsprechend ausgebildete und programmierte Steuervorrichtung bei der Unterdruck erzeugenden Einrichtung, insbesondere in Abhängigkeit weiterer Parameter, realisiert werden kann.

Zum Anlegen von Unterdruck und vorzugsweise auch zum Absaugen von Körperflüssigkeiten ist eine vorzugsweise flexible Saugleitung, beispielsweise in Form eines Drainageschlauchs, vorgesehen, der einenends über einen sogenannten Port im Bereich des Wundabdeckmaterials mit der Wundumgebung oder dem Wundraum und anderenends mit dem eingangs erwähnten Behälter zur Aufnahme von Körperflüssigkeiten bzw. mit der Unterdruck erzeugenden Einrichtung kommuniziert.

Neben der Unterdruckwundbehandlung sind auch andere Anwendungen der hier in Rede stehenden Vorrichtung zur Bereitstellung von Unterdruck für medizinische Anwendungen denkbar, insbesondere die Absaugung an sich beliebiger Körperflüssigkeiten, im Bereich der medizinischen Inkontinentenversorgung, der Versorgung von Stoma-Patienten oder im Bereich der Absaugung von Wundsekreten, gegebenenfalls unter Verwendung von Spülflüssigkeiten, auch ohne Anlegen eines Unterdrucks über wesentliche Zeiträume.

Der vorliegenden Erfindung liegt die Aufgabe zugrunde, eine Vorrichtung der vorausgehend beschriebenen Art dahingehend zu verbessern, dass sie in noch benutzerfreundlicherer Weise handhabbar und bedienbar ist.

Diese Aufgabe wird durch eine Vorrichtung mit den Merkmalen des Anspruchs 1 gelöst. Die scheibenförmige Ausbildung der beiden Gehäuseteile bedeutet im Sinne der vorliegenden Erfindung, dass die Gehäuseteile, wenn sie in einem stationären Betrieb mit ihrer Standseite bestimmungsgemäß auf einer horizontalen Unterlage abgestellt sind, jeweils eine Breite und Höhe aufweisen, die gegenüber einer Tiefe in horizontaler Richtung betrachtet größer sind. Die Gehäuseteile liegen also, wenn sie lösbar gegeneinander fixiert sind, mit ihren in der Scheibenebene liegenden Seiten gegeneinander an. Wenn vorausgehend von einer Trennebene die Rede ist, so bedeutet dies aber nicht zwangsläufig, dass die Gehäuseteile über eine exakte ebene Fläche gegeneinander anliegen. Vielmehr können in vorteilhafter Weise als Zentriermittel wirkende Vorsprünge oder dergleichen Formgebung in vorzugsweise komplementärer Form zueinander bei den Gehäuseteilen vorgesehen sein. Erfindungsgemäß werden die beiden Gehäuseteile also nicht ineinander gestülpt oder übereinander gestapelt, sondern sie liegen über die genannte im Wesentlichen vertikal orientierte Trennebene nebeneinander und gegeneinander an, wenn die Vorrichtung bestimmungsgemäß mit ihrer Standseite auf einer horizontalen Oberfläche abgestellt ist oder am Körper eines aufrecht stehenden Menschen getragen wird.

Dadurch dass der den zweiten Gehäuseteil bildende Behälter zusammen mit dem zweiten Gehäuseteil im mobilen Betrieb der Vorrichtung körperzugewandt und der die Unterdruck erzeugende Einrichtung aufnehmende erste Gehäuseteil körperabgewandt orientiert ist, ist es möglich, den zweiten Gehäuseteil gewissermaßen mittels des ersten Gehäuseteils abzudecken. Auf diese Weise könnte der zweite Gehäuseteil oder der Behälter auch durchsichtig ausgebildet werden, ohne dass eine unmittelbare Einsichtnahme durch dritte Personen möglich wäre. Des Weiteren gewährt die Anordnung des zweiten Gehäuseteils im Bereich des Körpers größere Gestaltungsmöglichkeiten zur Anpassung an den Körper als dies bei dem ersten die Unterdruck erzeugende Einrichtung aufnehmenden Gehäuseteil der Fall wäre. Weiter kann durch die körperabgewandte Anordnung des ersten Gehäuseteils der Zugang zu Bedienelementen für die Unterdruck erzeugende Einrichtung und deren Steuerung im Bereich der Sichtseite der Vorrichtung vorgesehen werden. Hierdurch ist auch der Zugriff durch den Benutzer der Vorrichtung selbst möglich, indem die Bedienkomponenten und möglicherweise auch Anzeigekomponenten dann körperabgewandt und vorzugsweise von oben einsehbar angeordnet sind.

Es erweist sich weiter als vorteilhaft, dass beim Fügen des ersten und zweiten Gehäuseteils zugleich eine Unterdruckkommunikation zwischen Behälter und Unterdruck erzeugender Einrichtung hergestellt wird, so dass der Fügevorgang insgesamt benutzerfreundlich wird und der Benutzer nicht in Kontakt mit Körperflüssigkeiten gelangt. In entsprechender Weise kommt diesem Gedanken beim Lösen der Gehäuseteile nach Gebrauch noch größere Bedeutung zu.

Um die beiden Gehäuseteile sicher aneinander zu halten, erweist es sich weiter als vorteilhaft, dass sie durch schnappende, rastende oder in sonstiger Weise formschlüssig wirkende Verriegelungs- oder Hintergriffsmittel gegeneinander lösbar fixierbar sind.

In weiterer Ausbildung der Erfindung hat es sich als besonders vorteilhaft erwiesen, wenn der zweite Gehäuseteil auf seiner dem Körper des Benutzers zugewandten Seite in Richtung auf den Körper des Benutzers in wenigstens einer vorzugsweise horizontalen Ebene konkav ausgebildet ist, so dass er ergonomisch an den Körper anlegbar ist. Die Vorrichtung lässt sich so beispielsweise im Hüftbereich in für den Benutzer bequemer Weise am Körper tragen. Die genannte Ebene verläuft in diesem Fall bei stehendem Benutzer/Patienten im Wesentlichen horizontal und die Schnittlinie dieser Ebene mit der körperzugewandten Seite des zweiten Gehäuseteils verläuft dann konkav. Es hat sich hierfür wenigstens abschnittsweise ein Krümmungsradius von 300 bis 500 mm, insbesondere von 300 bis 400 mm als vorteilhaft erwiesen.

Weiter kann es sich als vorteilhaft erweisen, wenn der zweite Gehäuseteil auf seiner dem Körper des Benutzers zugewandten Seite wenigstens bereichsweise in einer horizontalen und in einer vertikalen Ebene konkav ausgebildet ist. Auch dies kann beispielsweise die Anordnung der Vorrichtung im Hüftbereich eines Benutzers erleichtern. Für eine konkave Ausbildung in einer vertikalen Ebene erweist sich ein Krümmungsradius von wenigstens abschnittsweise 500 bis 900 mm, insbesondere von 600 bis 800 mm, insbesondere von 700 bis 800 mm als vorteilhaft.

Es wäre aber beispielsweise auch eine Ausführungsform denkbar, bei der der zweite Gehäuseteil auf seiner dem Körper zugewandten Seite in einer horizontalen Ebene konkav und in einer vertikalen Ebene abschnittsweise konvex ausgebildet ist, so dass insgesamt eine Sattelform realisiert wird.

Dessen ungeachtet kann es sich als vorteilhaft erweisen, wenn der zweite Gehäuseteil auf seiner dem Körper des Benutzers zugewandten Seite in einem von der Standseite der Vorrichtung abgewandten Bereich (also oben) eine Abschrägung weg vom Körper des Benutzers in Richtung auf den ersten Gehäuseteil aufweist. Die genannte Abschrägung kann hierbei in vorteilhafter Weise ergonomisch verrundet ausgebildet sein, was aber keinesfalls erforderlich ist. Auf diese Weise liegt die Vorrichtung mit einer etwas kleineren Fläche des zweiten Gehäuseteils gegen den Körper des Benutzers an, was es ihm aber ermöglicht, seinen Körper auch über die Vorrichtung zu beugen. In entsprechender Weise kann es sich als vorteilhaft erweisen, wenn der zweite Gehäuseteil auf seiner dem Körper des Benutzers zugewandten Seite im Übergang zu Seitenwandungen des zweiten Gehäuseteils oder zu Seitenwandungen des ersten Gehäuseteils abgeschrägt ausgebildet ist.

Im Hinblick auf ein benutzerfreundliches Fügen des ersten und des zweiten Gehäuseteils erweist es sich als vorteilhaft, wenn der erste und der zweite Gehäuseteil an ihren einander zugewandten Seiten Zentriermittel aufweisen und die Gehäuseteile im Wesentlichen quer zu der Trennebene gegeneinander fügbar sind. Die genannten Zentriermittel können in an sich beliebiger Weise etwa durch stift- oder konusförmige Elemente oder durch vorstehende und zurückgesetzte Gehäusebereiche gebildet sein, die insbesondere auch block- oder kastenförmig ausgebildet sein können und vorzugsweise komplementär und mit entsprechenden Anlaufschrägen versehen miteinander in einem zentrierenden Sinn zusammenwirken. Vorzugsweise sind diese Zentriermittel bzw. die gegeneinander anliegenden Seiten der Gehäuseteile so ausgebildet, dass sie sich nur in der korrekten Weise fügen lassen.

Zum Fügen der Gehäuseteile erweist es sich als vorteilhaft, wenn der eine Gehäuseteil von oben leicht schräg zur Vertikalen auf ein Auflager des anderen Gehäuseteils aufsetzbar und dann im wesentlichen quer zu der Trennebene in Anlage gegen den anderen Gehäuseteil schwenkbar ist.

Nach einer Ausführungsform der Erfindung erweist es sich als vorteilhaft, dass das Verriegelungs- oder Hintergriffsmittel von der Trennebene eines der Gehäuseteile vorsteht und beim Fügen der Gehäuseteile quer zur Fügerichtung der Gehäuseteile auslenkbar und in eine Hintergriffsstellung bringbar ist.

Das Verriegelungs- oder Hintergriffsmittel ist in weiterer Ausbildung der Erfindung durch ein manuell bedienbares Betätigungsorgan in Freigaberichtung bringbar. Das manuell bedienbare Betätigungsorgan kann grundsätzlich an beliebiger Stelle der Vorrichtung angeordnet sein. Es erweist sich jedoch als vorteilhaft, wenn es an einer Oberseite der Vorrichtung vorgesehen und insbesondere als eindrückbarer Taster ausgebildet ist. Auf diese Weise ist es nämlich möglich, dass die Vorrichtung auf einer vorzugsweise horizontalen Unterlage abgestellt wird und dann das Betätigungsorgan erfolgreich betätigt werden kann, ohne dass die Vorrichtung seitlich wegrutscht.

Zum Halten, Anheben oder Handhaben der Vorrichtung als Ganzes und/oder nur des einen oder anderen Gehäuseteils erweist es sich als vorteilhaft, wenn der eine oder andere Gehäuseteil für einen manuellen Eingriff ausgebildet ist, so dass die Vorrichtung oder nur der betreffende Gehäuseteil hierdurch ergriffen und von dem anderen Gehäuseteil gelöst werden kann. Es erweist sich als besonders vorteilhaft, wenn der zweite Gehäuseteil für diese Zwecke eine Eingriffsmulde oder Eingriffsvertiefung aufweist. Diese Eingriffsmulde oder Eingriffsvertiefung ist vorzugsweise im Bereich eines manuell bedienbaren Betätigungsorgans für ein Verriegelungs- oder Hintergriffsmittel vorgesehen, so dass ein Benutzer oder eine Pflegeperson mit nur einer Hand den zweiten Behälterteil entriegeln und vom ersten Behälterteil lösen kann. Diese Eingriffsmulde oder Eingriffsvertiefung ist vorzugsweise in einem oberen und vom Körper weg geneigten Bereich des zweiten Gehäuseteils auf der dem Körper zugewandten Seite des zweiten Gehäuseteils vorgesehen. Auf diese Weise ist es einem Benutzer ohne weiteres möglich, ausgehend vom mobilen Betrieb, die Vorrichtung mit einer Hand zu ergreifen und mit der anderen Hand ein später zu erörterndes insbesondere gurtartiges Befestigungsmittel zu lösen.

Im Hinblick auf eine hohe Standfestigkeit der Vorrichtung im stationären Betrieb oder auch beim zeitweiligen Absetzen der Vorrichtung auf einer vorzugsweise ebenen Fläche erweist es sich als vorteilhaft, wenn ein Schwerpunkt der Vorrichtung (im noch ungebrauchten Zustand des Behälters) in der unteren Hälfte der Vorrichtung, also unterhalb einer gedachten Horizontalmittelebene auf halber Höhe der Vorrichtung liegt.

Im Hinblick darauf erweist es sich auch als vorteilhaft, wenn das Verhältnis einer Standfläche der Vorrichtung zu ihrer Höhe 4,0 - 6,0 m²/m, insbesondere 4,5 - 5,5 m²/m beträgt. Die Standfläche der Vorrichtung kann beispielsweise ca. 81 cm² und ihre Höhe ca. 16 cm betragen.

Es wäre denkbar und für viele Anwendungen vorteilhaft, wenn die Standfläche der Vorrichtung im aneinander gefügten Zustand ihrer beiden Gehäuseteile von beiden Gehäuseteilen gebildet ist. Nach einer weiteren Ausführungsform der Erfindung hat es sich jedoch als hinreichend erwiesen, wenn die Standfläche der Vorrichtung nur von dem ersten Gehäuseteil gebildet ist. Hierfür erweist es sich als vorteilhaft, wenn das erste Gehäuseteil einen in eine Formausnehmung des ersten Gehäuseteils vorspringenden Bereich aufweist, der einen Teil der Standfläche bildet. Auf diese Weise wird nämlich erreicht, dass die Standfestigkeit des die Unterdruck erzeugende Einrichtung nebst Steuerkomponenten aufnehmenden ersten Gehäuseteils in quasi unveränderter Form auch nach dem Abnehmen des zweiten Gehäuseteils gegeben ist.

Nach einem weiteren Erfindungsgedanken wird vorgeschlagen, die Vorrichtung so auszubilden, dass der erste Gehäuseteil den zweiten Gehäuseteil in frontaler Blickrichtung auf eine körperabgewandte Sichtseite des ersten Gehäuseteils vorzugsweise vollständig oder zu wenigstens 90% der Aufsichtsfläche in dieser Blickrichtung verdeckt. Dies eröffnet wiederum weitere Möglichkeiten, den zweiten Gehäuseteil preiswert auszubilden, da er nur zum geringen Teil im Bereich seiner seitlichen Flächen für den Benutzer oder für Dritte einsehbar ist, wenn die Vorrichtung am Körper des Benutzers getragen wird. Der Behälter muss also nicht mehr in Tragebeuteln "versteckt" werden.

Nach einem Erfindungsgedanken von besonderer Bedeutung wird vorgeschlagen, dass die Vorrichtung gewissermaßen als Sachgesamtheit mehrere zweite Gehäuseteile mit einem jeweiligen Behälter umfasst, die wahlweise zur Bildung der Vorrichtung mit dem ersten Gehäuseteil verbindbar sind, wobei die zweiten Gehäuseteile und deren Behälter unterschiedliche Größe, insbesondere in Tiefenrichtung, also in Richtung auf den Benutzer, aufweisen. Es können auf diese Weise unterschiedliche Aufnahmekapazitäten durch die verschiedenen Gehäuseteile und deren Behälter bereitgestellt werden. Hierbei können Gehäuseteile vorgesehen werden, die sich für einen mobilen Betrieb eignen und solche, die eher für einen stationären Betrieb bestimmt sind, also insbesondere großvolumiger ausgebildet sind, damit die Behälter zur Aufnahme von Flüssigkeit weniger häufig ausgetauscht werden müssen.

Um Kontamination und Verstopfung der Unterdruck erzeugenden Einrichtung zu verhindern, erweist es sich als vorteilhaft, wenn zwischen dem Behälter und der Unterdruck erzeugenden Einrichtung ein Filtermittel vorgesehen ist, welches insbesondere zusammen mit dem Behälter nach Gebrauch als Einwegartikel wegwerfbar ist.

Weiter erweist es sich als vorteilhaft, wenn eine Füllstandsanzeigeeinrichtung für den Behälter vorgesehen ist. Diese Füllstandsanzeigeeinrichtung kann beispielsweise durch eine hinreichend durchsichtige Ausbildung einer Gehäusewandung des zweiten Gehäuseteils verwirklicht sein, oder sie kann in sonstiger, an sich beliebiger Weise, insbesondere über elektronische Sensoren und Anzeigemittel gebildet sein. Weiter erweist sich eine Überwachungseinrichtung als vorteilhaft, welche in Abhängigkeit von dem erreichten Füllstand im Behälter eine Alarmmeldung ausgibt. Der Behälter des zweiten Gehäuseteils kann ein superabsorbierendes Medium enthalten, um die angesaugte Flüssigkeit zu binden und ein Schwappen der Flüssigkeit zu verhindern.

Es wurde bereits darauf hingewiesen, dass die erfindungsgemäße Ausbildung der Vorrichtung es in vorteilhafter Weise gestattet, dass an einer körperabgewandten Sichtseite des ersten Gehäuseteils Bedienelemente und Anzeigeelemente für die Unterdruck erzeugende Einrichtung vorgesehen sind. In vorteilhafter Weise sind die Bedienelemente in Form eines Touchscreens ausgebildet.

Nach einem unabhängigen Erfindungsgedanken erweist es sich als vorteilhaft, wenn ein Neigungssensor bei dem ersten Gehäuseteil vorgesehen ist, der über eine Steuerungseinrichtung bei einer vorgegebenen Neigung des ersten Gehäuseteils zur Vertikalen veranlasst, dass die Anzeige auf einem Anzeigeelement um vorzugsweise 180° gedreht wird, so dass sie von oben durch den Benutzer ablesbar ist. Ausgehend von der normalen vertikalen Anordnung der Vorrichtung, wenn diese beispielsweise stationär betrieben oder von einem aufrecht stehenden Benutzer im mobilen Betrieb getragen wird, braucht der Benutzer die Vorrichtung lediglich bis zu einer vorgegebenen Neigung zu neigen, insbesondere um 45°, insbesondere um 40°, insbesondere um 30°, so dass sich die Anzeige auf dem Anzeigeelement (Display) dreht und der Benutzer die Anzeige von oben ablesen kann.

Nach einem weiteren Erfindungsgedanken von wesentlicher Bedeutung erweist es sich als vorteilhaft, dass im Wesentlichen die gesamte körperabgewandte Sichtseite des ersten Gehäuseteils von einer flächenhaften Abdeckung gebildet oder überfangen ist, so dass schmutzaufnehmende Fugen im Bereich der Bedienelemente vermieden werden.

Weiter erweist es sich als vorteilhaft, wenn an dem ersten Gehäuseteil eine Schnittstelle für Datenaustausch, insbesondere eine USB-Schnittstelle, vorgesehen ist, die insbesondere in der Trennebene zum zweiten Gehäuseteil vorgesehen und so durch das zweite Gehäuseteil verdeckt und insbesondere gegen Verschmutzen geschützt ist. Auf diese Weise können programmtechnische Steuerinformationen an eine elektronische Steuereinrichtung der Vorrichtung übergeben werden.

Die schon erwähnten Befestigungsmittel zum Befestigen der Vorrichtung am Körper des Benutzers für den mobilen Betrieb der Vorrichtung umfassen in vorteilhafter Weise einen flexiblen streifenförmigen oder gurtförmigen Abschnitt oder mehrere solcher Abschnitte, der oder die an gegenüberliegenden Seiten des ersten und/oder zweiten Gehäuseteils lösbar befestigbar ist oder sind, also für den stationären Betrieb der Vorrichtung von den Gehäuseteilen gelöst werden kann oder können. Es kann sich bei den Befestigungsmitteln aber auch um auf der Außenseite der Gehäuseteile zu liegen kommende Bügel oder Laschen handeln, an die weitere Befestigungsmittel lösbar angefügt werden können, z.B. Gürtelabschnitte, Trageriemen oder dergleichen.

Die Befestigungsmittel sind in vorteilhafter Weise zwischen den gegeneinander anliegenden Gehäuseteilen lösbar angeordnet. Sie sind insbesondere in einen Einsteckschlitz zwischen den gegeneinander anliegenden Gehäuseteilen einsteckbar oder an an der Außenseite der Gehäuseteile vorgesehene Bügel, Laschen oder dergleichen lösbar ankoppelbar.

Die vorzugsweise flexiblen Befestigungsmittel werden in bevorzugter Weise nach Art eines Gürtels um den Körperumfang, vorzugsweise im Hüftbereich oder jedenfalls grundsätzlich auch um den Oberschenkel, Oberarm oder Thorax herum gelegt und halten so die Vorrichtung am Körper. In besonders bevorzugter Weise sind die Befestigungsmittel mit dem ersten und/oder zweiten Gehäuseteil der Vorrichtung nach Art eines Schnellverschlusses verbindbar und auch wieder lösbar. Die Befestigungsmittel können an ihren freien Enden in die Gehäuseteile einsteckbare Schnellverschlussmittel, insbesondere mit federnden Hintergriffsmitteln, oder einsteckbare oder in sonstiger Weise befestigbare Bügel für das Festlegen von flexiblen Befestigungsmitteln aufweisen. Nach einer bevorzugten Ausführungsform sind die Befestigungsmittel zwischen dem ersten und dem zweiten Gehäuseteil, also in der Trennebene zwischen den Gehäuseteilen lösbar angeordnet. Es wäre eine Ausführungsform denkbar und vorteilhaft, bei der die Befestigungsmittel durch Trennen der Gehäuseteile voneinander abgenommen werden können. Oder es wäre eine Ausführungsform denkbar und vorteilhaft, bei der das Lösen der Befestigungsmittel unabhängig von einem Trennen der Gehäuseteile voneinander möglich ist. Es kann auch ein zusätzlicher Schultergurt oder -riemen vorgesehen sein, der alternativ oder zusätzlich zu einem Hüftgürtel verwendet werden kann. Er kann zum Beispiel mittels eines Karabinerverschlusses oder eines an sich beliebigen Schnellverschlusses an einem Bügel oder an einer Lasche bei den Gehäuseteilen lösbar festgelegt werden.

Weiter erweist es sich als vorteilhaft, wenn die Gehäuseteile im gefügten Zustand der Vorrichtung mit Ausnahme des Anschlusses für die Saugleitung oder einen Mess- oder Spülkanal und einer etwaigen Eingriffsmulde oder Eingriffsvertiefung keine hintergreifbaren Komponenten an der Außenseite aufweisen.

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen der erfindungsgemäßen Vorrichtung. Für die Merkmale der Patentansprüche wird jeweils separat und losgelöst von einer Rückbeziehung der Ansprüche in beliebiger Kombination Schutz in Anspruch genommen. In der Zeichnung zeigt:
- Figuren 1 a bis e: verschiedene Ansichten einer bevorzugten Ausführungsform der erfindungsgemäßen am Körper tragbaren Vorrichtung zur Bereitstellung von Unterdruck für medizinische Anwendungen;
- Figuren 2a bis e: verschiedene Ansichten eines eine Unterdruck erzeugende Einrichtung und Steuerungskomponenten umfassenden ersten Gehäuseteils der Vorrichtung nach Figur 1;
- Figuren 3a bis i: verschiedene Ansichten eines einen Behälter zur Aufnahme von Körperflüssigkeiten bildenden zweiten Gehäuseteil der Vorrichtung nach Figur 1;
- Figuren 4a bis e: den Figuren 1a bis e entsprechende Ansichten einer weiteren Ausführungsform der erfindungsgemäßen Vorrichtung, wobei der zweite Gehäuseteil größer dimensioniert ist als bei der Vorrichtung nach Figuren 1a bis e; und
- Figur 5: eine Schnittansicht durch die Vorrichtung im Bereich der Unterdruckkommunikation zwischen dem ersten und zweiten Gehäuseteil;
- Figuren 6, 7: eine Ansicht der zweiten Gehäuseteile der Vorrichtungen nach Figuren 1 und 4 von unten zur Verdeutlichung der unterschiedlichen Standflächen dieser zweiten Gehäuseteile.

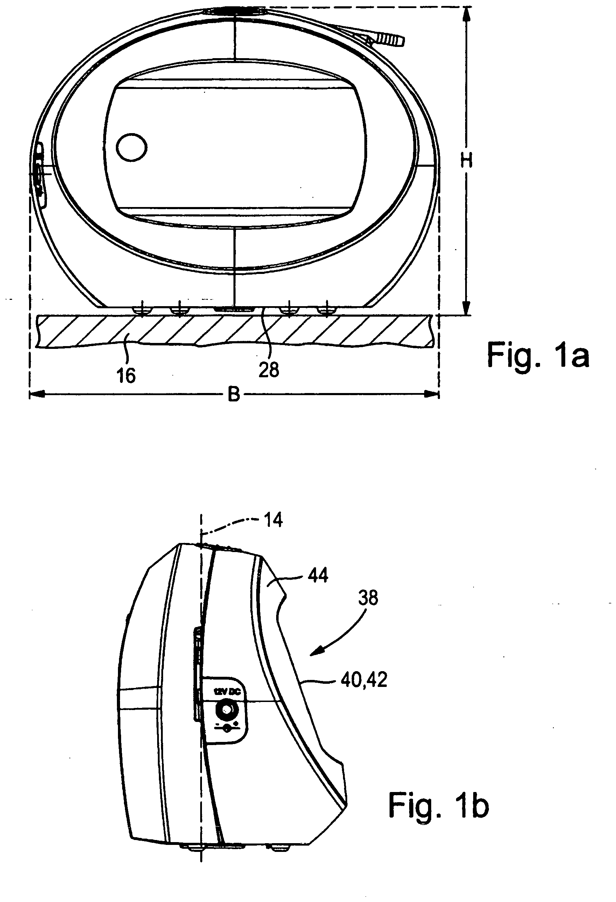

Die Figuren 1a bis e zeigen eine erste Ausführungsform einer erfindungsgemäßen tragbaren Vorrichtung 2 zur Bereitstellung von Unterdruck für medizinische Anwendungen. Die Vorrichtung umfasst einen ersten Gehäuseteil 4, in dem eine Unterdruck erzeugende Einrichtung in Form einer Luftpumpe sowie elektrische und elektronische Steuerkomponenten für die Vorrichtung insgesamt aufgenommen sind, einschließlich Batterien oder vorzugsweise wiederaufladbare Akkus. Ein Ladeanschluss für die Akkus ist mit Bezugszeichen 6 bezeichnet. Des Weiteren umfasst die Vorrichtung 2 ein zweites Gehäuseteil 8, welches im bevorzugten Fall zugleich einen Behälter 10 zur Aufnahme von Körperflüssigkeiten, insbesondere zur Aufnahme von aus einer Wunde abgesaugten Wundsekreten, bildet. Bevorzugtermaßen ist der gesamte zweite Gehäuseteil 8 als wegwerfbarer Einmalartikel ausgebildet. An seinem oberen Bereich ist ein Anschlussstutzen 12 für eine nicht dargestellte Saugleitung vorgesehen, die dann beispielsweise bei der Verwendung der Vorrichtung 2 zur Unterdrucktherapie von Wunden zu einem die Wunde druckdicht verschließenden Wundverband führt und dort beispielsweise über einen Port mit dem Wundraum kommuniziert, um im Wundraum einen Unterdruck anzulegen und aufrechtzuerhalten und Wundsekrete in den Behälter 10 abzusaugen. Hierfür kommuniziert der Behälter 10 mit der Unterdruck erzeugenden Einrichtung. Weiter ist ein Anschluss 13 für einen optionalen Mess- oder Spülkanal, der wie die Saugleitung zur Wunde führt, dargestellt. Dieser Anschluss durchgreift den zweiten Gehäuseteil 8 und mündet in den ersten Gehäuseteil 4, von wo aus der Mess- oder Spülkanal beispielsweise mit Luft als Spülmedium beaufschlagt werden kann und/oder ein Druck in diesem Mess- oder Spülkanal detektiert und ausgewertet werden kann.

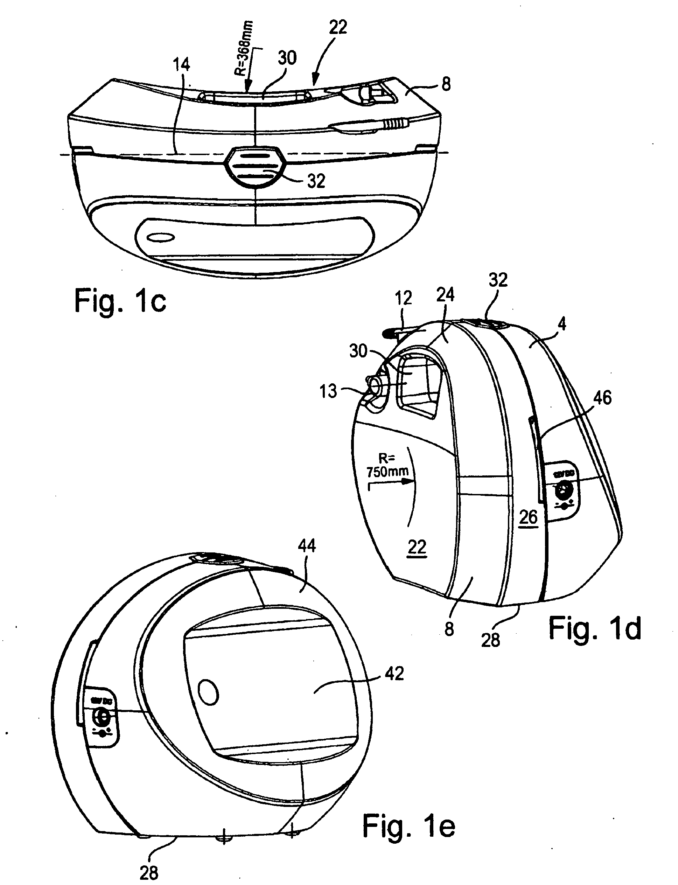

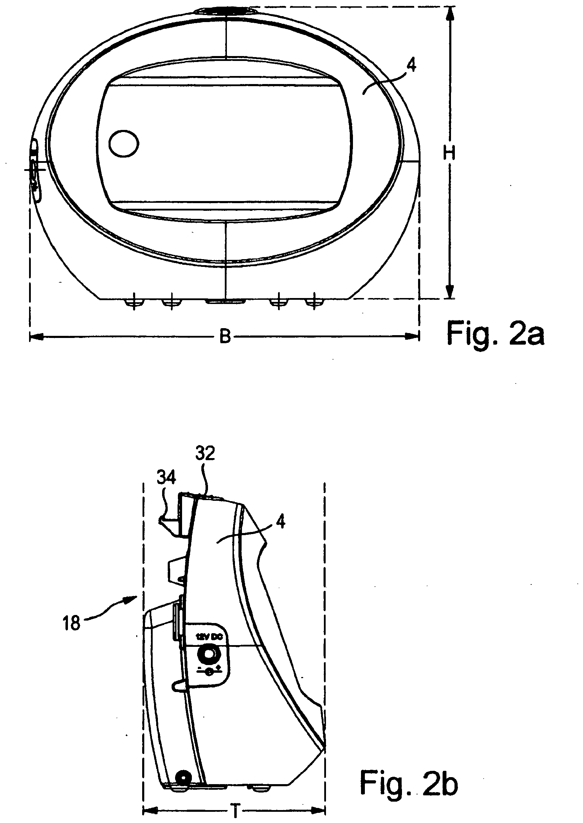

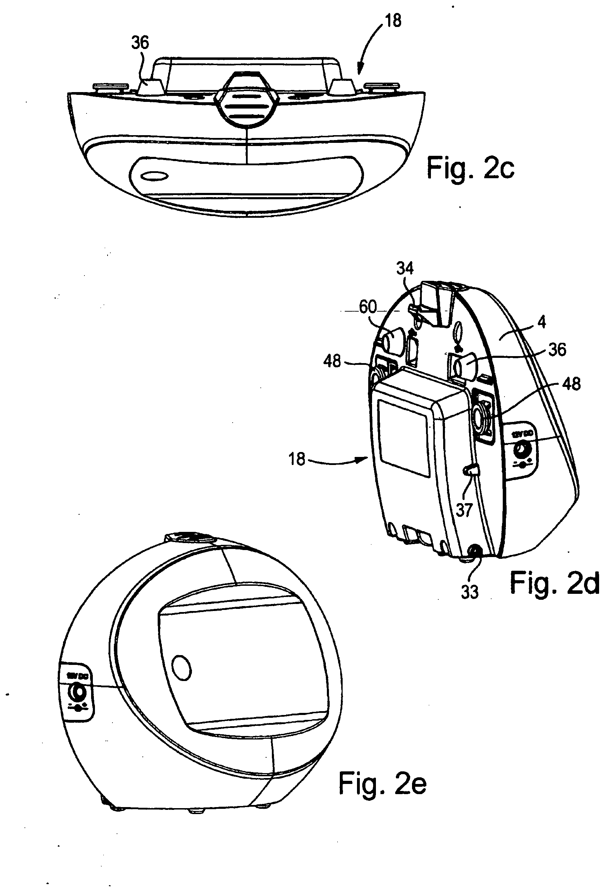

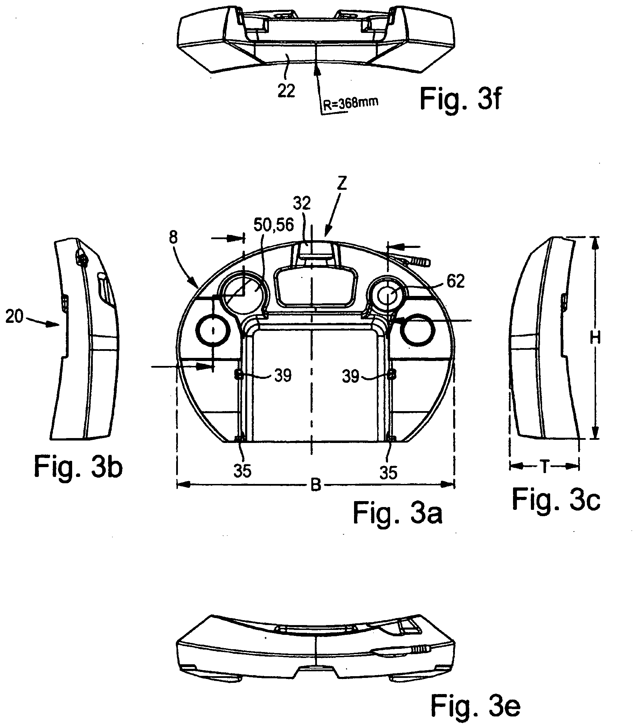

Erfindungsgemäß liegen die Gehäuseteile 4 und 8 über eine im Wesentlichen vertikale Trennebene 14, die in verschiedenen Figuren angedeutet ist, gegeneinander an. Wenn die Vorrichtung 2, wie in Figur 1a angedeutet, auf einer ebenen horizontalen Unterlage 16 abgestellt ist, so ist die Trennebene 14 im Wesentlichen vertikal orientiert. Dies bedeutet, dass die beiden Gehäuseteile 4, 8 nicht ineinander eingesetzt oder übereinander gestapelt sind, sondern dass sie nebeneinander im bestimmungsgemäß zusammengesetzten Zustand der Vorrichtung 2 zu liegen kommen. Der Begriff der Trennebene 14 ist also nicht in dem Sinn zu verstehen, dass es sich um eine geometrisch ebene Fläche handeln muss, was unmittelbar aus den Figuren 2a bis e ersichtlich wird, welche den ersten Gehäuseteil 4 in verschiedenen Ansichten zeigen. Man erkennt sofort, dass die dem zweiten Gehäuseteil 8 zugewandte Seite 18 des ersten Gehäuseteils 4 keinesfalls eben, sondern mit einer Vielzahl von in Richtung auf den zweiten Gehäuseteil 8 vorspringenden Elementen ausgebildet ist. Die dem ersten Gehäuseteil 4 zugewandte Seite 20 des zweiten Gehäuseteils 8 ist im Wesentlichen komplementär zu der Ausbildung der Seite 18 des ersten Gehäuseteils 4 ausgebildet, so dass die beiden Gehäuseteile 4, 8 nur in einer korrekten Weise miteinander gefügt bzw. aneinander befestigt werden können. Die beiden Gehäuseteile 4, 8 sind insgesamt scheibenförmig ausgebildet, d. h. ihre Breite B in horizontaler Richtung und ihre Höhe H in vertikaler Richtung sind jeweils größer als ihre Tiefe T in horizontaler Richtung und senkrecht zur Breitenerstreckung. Hierdurch ist es möglich, dass die Vorrichtung 2 in Tiefenrichtung insgesamt so ausgebildet und bemessen werden kann, dass sie bequem am Körper eines Benutzers getragen werden kann. Erfindungsgemäß ist die Vorrichtung 2 so ausgebildet, dass die nebeneinander angeordneten Behälterteile 4, 8 derart am Körper positionierbar sind, dass der zweite Behälterteil 8 körperzugewandt, also zwischen dem Körper und dem ersten Gehäuseteil 4, zu liegen kommt und der erste Gehäuseteil 4 körperabgewandt zu liegen kommt, also im Wesentlichen die Sichtseite der Vorrichtung 2 bildet. Daher ist die dem Körper des Benutzers zugewandte Seite 22 des zweiten Gehäuseteils 8 verrundet ausgebildet. Wie sich den Figuren 1c, 1d, 3f, 3e entnehmen lässt, ist die körperzugewandte Seite 22 im Schnitt mit einer horizontalen Ebene betrachtet konkav ausgebildet und umfasst im beispielhaft dargestellten Fall abschnittsweise einen Krümmungsradius R von beispielhaft 368 mm (Figur 1c, 3f). Zusätzlich ist die körperzugewandte Seite 22 im Schnitt mit einer vertikalen Ebene betrachtet ebenfalls konkav ausgebildet und weist dort einen Krümmungsradius R von beispielhaft 750 mm auf (Figur 1d). Auf diese Weise lässt sich die Vorrichtung 2 ergonomisch im Hüftbereich eines Benutzers anordnen und tragen.

Man erkennt des Weiteren, dass der zweite Gehäuseteil 8 auf seiner körperzugewandten Seite 22 in einem oberen Bereich und auch seitlich eine Abschrägung 24 weg vom Körper des Benutzers in Richtung auf den ersten Gehäuseteil 4 bzw. in Richtung auf Seitenwandungen 26 oder eine Umfangsstirnseite der Scheibenform des zweiten Gehäuseteils 8 hin umfasst. Die Abschrägung 24 ist im beispielhaft dargestellten Fall umlaufend ausgebildet; sie erstreckt sich ausgehend von der Standseite 28 von unten nach oben, verläuft dort bogenförmig zur anderen Seite und dann wieder nach unten zur Standseite 28 zurück.

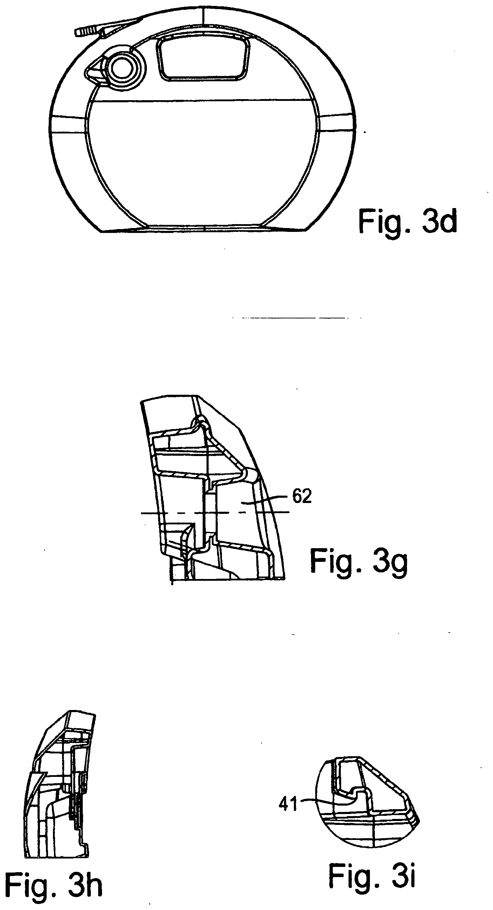

Man erkennt des Weiteren aus den Figuren 1d und 3, dass auf der körperzugewandten Seite 22 des zweiten Gehäuseteils 8 eine Eingriffsvertiefung 30 in Form einer durch den zweiten Gehäuseteil 8 hindurch gehenden Öffnung ausgebildet ist, und zwar in einem oberen leicht vom Körper weg geneigten Bereich des zweiten Gehäuseteils 8. Hierdurch kann die Vorrichtung 2 insgesamt oder nur deren zweiter Gehäuseteil 8 mit einer Hand ergriffen und gehandhabt werden.

Bei der dargestellten bevorzugten Ausführungsform ist nahe bei dieser Eingriffsvertiefung 30 in einer Oberseite der Vorrichtung 2 ein manuell bedienbares Betätigungsorgan 32, beispielsweise in Form eines Tasters, vorgesehen, der auf ein Verriegelungs- oder Hintergriffsmittel 34 einwirkt (s. Figuren 2b und 2d). Im gefügten Zustand der beiden Gehäuseteile 4 und 8 befindet sich das Verriegelungs- oder Hintergriffsmittel 34 in einem die beiden Gehäuseteile 4, 8 formschlüssig aneinander haltenden verriegelten Zustand. Erst durch Betätigen des Betätigungsorgans 32 wird die Verriegelung gelöst, so dass die Gehäuseteile 4, 8 voneinander separiert werden können. Durch die Anordnung und Ausbildung der Eingriffsvertiefung 30 und des manuell bedienbaren Betätigungsorgans 32 in räumlicher Nähe zueinander und derart, dass ein Benutzer sowohl in die Eingriffsvertiefung 30 eingreifen als auch mit einem Finger derselben Hand gleichzeitig das Betätigungsorgan 32 zu bedienen vermag, wird eine Einhandbedienung zum Lösen des zweiten Gehäuseteils 8 vom ersten Gehäuseteil 4 realisiert. Dies erweist sich als besonders vorteilhaft, da solchenfalls ein mit Körperflüssigkeiten befüllter zweiter Gehäuseteil 8 mit nur einer Hand gelöst und in ein Entsorgungsbehältnis gegeben werden kann.

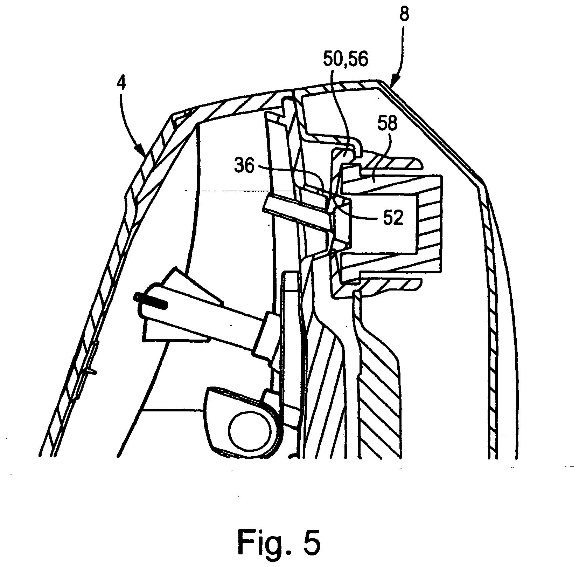

Zum Fügen der beiden Gehäuseteile 4, 8 wird der zweite Gehäuseteil 8 leicht schräg von hinten und von oben mit seinem unteren Rand auf zwei einen Drehpunkt bildende Zapfen 33 (Figur 2d) des ersten Gehäuseteils 4 aufgesetzt. In dem zweiten Gehäuseteil ist hierfür am unteren Rand ein ausgesparter Bereich 35 (Figur 3a) zur Aufnahme des Zapfens 33 ausgebildet. Wenn Zapfen 33 und ausgesparter Bereich 35 miteinander in Eingriff sind, so lässt sich der zweite Gehäuseteil 8 gegen den ersten Gehäuseteil 4 schwenken. Hierdurch werden die einander zugewandten Seiten 18, 20 gegeneinander gelegt und gelangen so selbstzentrierend (unterstützt durch weitere Führungs- oder Zentriermittel 37 (Figur 2d) und 39 (Figur 3a) und die komplementäre Ausbildung der einander zugewandten Seiten 18, 20 der Gehäuseteile 4,8) in ihre bestimmungsgemäße Position. Durch Gegeneinanderbewegen der beiden Gehäuseteile 4, 8, insbesondere im Wesentlichen quer zu der vertikalen Trennebene 14, wird das Verriegelungs- oder Hintergriffsmittel 34 selbsttätig ausgelenkt und rastet dann in seine die Gehäuseteile 4, 8 gegeneinander verriegelnde Stellung. Hierfür ist an dem zweiten Gehäuseteil 8 ein Rasthaken 41 (Figur 3i) vorgesehen, der von dem Verriegelungs- oder Hintergriffsmittel 34 untergriffen wird. Wenn die Gehäuseteile 4, 8 in ihre verriegelte Stellung gebracht werden, wird dann auch automatisch eine Unterdruckkommunikation zwischen dem Inneren des Behälters 10 des zweiten Gehäuseteils 8 und der Unterdruck erzeugenden Einrichtung über Anschlussmittel 36 hergestellt (später im Zusammenhang mit Figur 5 beschrieben).

Eine körperabgewandte Sichtseite 38 des ersten Gehäuseteils 4 ist leicht zur Vertikalen geneigt ausgebildet, so dass sich die Scheibenform nach oben hin verjüngt. Auf diese Weise ist eine leichtere Einsichtnahme auf die Sichtseite 38 gegeben. Dort sind Bedienelemente 40 und Anzeigeelemente 42 insbesondere in Form eines Touchscreens vorgesehen. Im Wesentlichen die gesamte Sichtseite 38 ist von einer flächenhaften Abdeckung 44 überfangen oder gebildet, so dass im Bereich der Bedienelemente 40 keine Schmutz aufnehmenden Fugen gebildet werden.

Des Weiteren zeigen die Figuren im Bereich der Trennebene 14 zwischen den gegeneinander anliegenden Gehäuseteilen 4, 8 einen Einsteckschlitz 46 zum Einstecken und lösbaren Fixieren eines Befestigungsmittels, insbesondere und vorzugsweise in Form eines flexiblen Gürtels, oder eines Bügels oder einer Lasche, an dem/der beispielsweise ein Gürtel oder ein Schultertragriemen befestigt werden kann, oder in sonstiger Form. Es erweist sich als vorteilhaft, dass dieses Befestigungsmittel von den Gehäuseteilen 4, 8 gelöst werden kann und somit nicht stört, wenn die Vorrichtung 2 im stationären Betrieb, also auf einer vorzugsweise ebenen Unterlage 16 stehend, benutzt wird, etwa wenn ein hiermit zu behandelnder Patient in einem Krankenbett ruht. In Figur 2d sind an der Seite 18 des ersten Gehäuseteils 4 Mittel 48 angedeutet, an denen die in den Einsteckschlitz 46 eingesteckten Befestigungsmittel festlegbar oder gehalten sind.

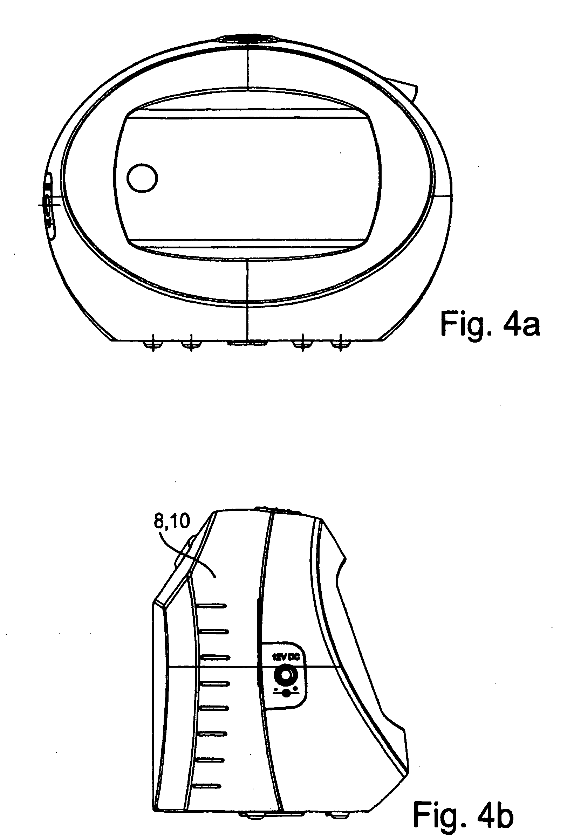

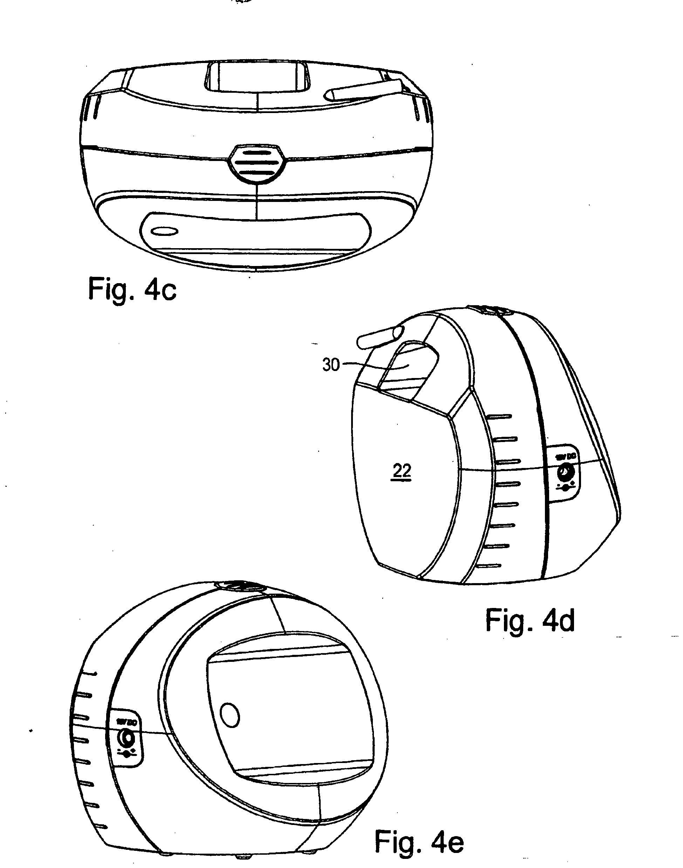

Die in Figuren 4a bis e dargestellte weitere Ausführungsform der erfindungsgemäßen Vorrichtung unterscheidet sich von der in Figuren 3 dargestellten Ausführungsform dadurch, dass der zweite Gehäuseteil 8 und der von ihm gebildete Behälter 10 großvolumiger ausgebildet ist. Die Abschrägung im oberen Bereich der körperzugewandten Seite 22 des zweiten Gehäuseteils 8, wo die Griffmulde 30 ausgebildet ist, ist noch etwas stärker vom Körper des Benutzers weg geneigt. Auf diese Weise ist ein noch besserer Zugriff möglich. Dieser größere zweite Gehäuseteil 8 eignet sich eher für einen stationären Betrieb der Vorrichtung 2; er könnte hierfür auch eine nach außen konvex gewölbte Seite 22 aufweisen oder gar noch ausladender ausgebildet sein als dies die Figuren 4 zeigen.

Figur 5 zeigt im Einzelnen die Ausbildung der Unterdruckkommunikation zwischen dem Inneren des den Behälter 10 bildenden zweiten Gehäuseteils 8 und dem ersten Gehäuseteil 4. Die Ansaugseite einer nicht dargestellten Unterdruck erzeugenden Einrichtung führt zu dem konisch ausgebildeten Anschlussmittel 36, welches sich konisch in Richtung auf den zweiten Gehäuseteil 8 verjüngt. Auf diese Weise kann gegen das konische Anschlussmittel 36 des ersten Gehäuseteils 4 ein wenigstens geringfügig nachgiebig ausgebildetes Gegenanschlussmittel 50 des zweiten Gehäuseteils 8 dichtend angelegt werden, welches im beispielhaft dargestellten Fall eine kreisförmige Öffnung 52 aufweist, die von einer nachgiebigen Dichtlippe 54 begrenzt ist. Dieses Gegenanschlussmittel 50 mündet in das Innere des zweiten Gehäuseteils 8. Es bildet zugleich ein Filteraufnahmemittel 56 für einen Filter 58, der im beispielhaft dargestellten Fall als topfförmiger Filter ausgebildet ist und verhindert, dass Bakterien in den ersten Gehäuseteil 4 eingesogen werden. Man erkennt ohne weiteres, dass beim Gegeneinanderbewegen der beiden Gehäuseteile 4, 8 das Anschlussmittel 36 des ersten Gehäuseteils 4 mit dem Gegenanschlussmittel 50 des zweiten Gehäuseteils 8 eine nach außen abgedichtete Druckkommunikation ausbildet.

In ähnlicher Weise ist die Kopplung zwischen dem Anschluss 13 für einen Mess- bzw. Spülkanal und dem zugehörigen ebenfalls beispielhaft konusförmig ausgebildeten Anschlussmittel 60 am ersten Gehäuseteil 4 ausgebildet. Wie aus Figur 3g ersichtlich ist, lässt sich in die Durchgangsöffnung 62 in dem zweiten Gehäuseteil 8 ein nicht dargestelltes Kopplungs- oder Tüllenteil einsetzen, welches dann den in Figur 1d dargestellten Anschluss 13 für den Mess- oder Spülkanal bildet. Dieses nicht dargestellte Kopplungs- oder Tüllenteil kann dann mit dem konusförmigen Anschlussmittel 60 druckdicht gekoppelt werden. Auf diese Weise kann über eine Leitung ein fluides Medium, insbesondere Luft oder eine Spülflüssigkeit, zur Wunde geleitet werden, um die Absaugung von Wundsekreten zu unterstützen. Typischerweise sind eine Mess- oder Spülleitung und die Saugleitung als wegwerfbare Einwegkomponenten Zubehörbestandteil zu dem zweiten Gehäuseteil; sie werden nach Gebrauch zusammen mit diesem entsorgt.

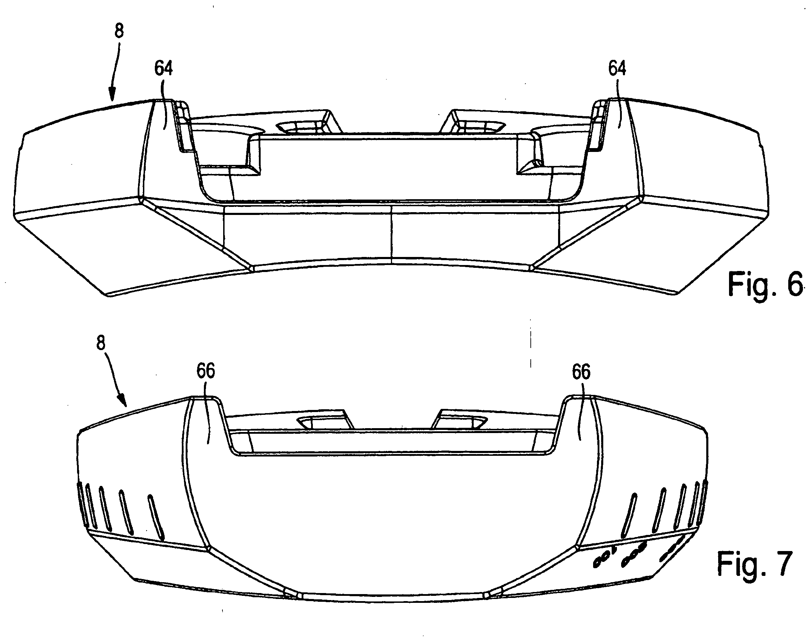

Schließlich verdeutlichen die Figuren 6 und 7 unterschiedlich große Standflächen 64 (mit ca. 7 cm²) und 66 (mit ca. 55 cm²) bei den verschieden großen zweiten Gehäuseteilen 8 der Ausführungsformen nach Figuren 1 und 4. Die Gehäuseteile 8 sind mit diesen Standflächen 64 bzw. 66 auf eine Unterlage abstellbar, wenn sie von dem ersten Gehäuseteil 4 abgenommen sind. Wenn sie am ersten Gehäuseteil 4 gehalten sind, so wird die Standfläche der Vorrichtung 2 von dem ersten Gehäuseteil 4 gebildet. Es können an den Standflächen der Gehäuseteile 4, 8 auch Fußelemente vorgesehen sein, wie dies aus den Figuren 1b, 2b, 4b ersichtlich ist.

## Patentansprüche

1. Am Körper eines Benutzers tragbare Vorrichtung (2) zur Bereitstellung von Unterdruck für medizinische Anwendungen, insbesondere zur Unterdruckbehandlung von Wunden am menschlichen oder tierischen Körper, mit einer Unterdruck erzeugenden Einrichtung und einem nach Gebrauch wegwerfbaren Einweg-Behälter (10) zur Aufnahme von Körperflüssigkeiten, insbesondere von aus einer Wunde abgesaugten Wundsekreten, und mit einem Anschluss (12) für eine hierfür zum Körper führende Saugleitung, wobei die Unterdruck erzeugende Einrichtung in oder an einem ersten Gehäuseteil (4) der Vorrichtung angeordnet ist und der Behälter (10) einen zweiten Gehäuseteil (8) der Vorrichtung bildet, und die Gehäuseteile (4, 8) lösbar gegeneinander fixierbar sind, und wobei die Vorrichtung (2) Befestigungsmittel aufweist, so dass sie am Körper des Benutzers tragbar und mitführbar ist, wobei beide Gehäuseteile (4, 8) im wesentlichen scheibenförmig ausgebildet sind und über eine im wesentlichen vertikal orientierte Trennebene (14) - gegeneinander anliegen, **dadurch gekennzeichnet, dass** der erste Gehäuseteil (4) im am Körper des Benutzers mitgeführten mobilen Zustand der Vorrichtung körperabgewandt und der zweite Gehäuseteil (8) körperzugewandt vorgesehen ist und dass beim Fügen des ersten und zweiten Gehäuseteils (4, 8) zugleich eine Unterdruckkommunikation zwischen Behälter (10) und Unterdruck erzeugender Einrichtung hergestellt wird und dass die Gehäuseteile (4, 8) durch schnappende, rastende oder in sonstiger Weise formschlüssig wirkende Verriegelungs- oder Hintergriffsmittel (34) gegeneinander lösbar fixierbar sind.

2. Vorrichtung nach Anspruch 1, **dadurch gekennzeichnet, dass** der zweite Gehäuseteil (8) auf seiner dem Körper des Benutzers zugewandten Seite (22) in Richtung auf den Körper des Benutzers in wenigstens einer vorzugsweise horizontalen Ebene konkav ausgebildet ist.

3. Vorrichtung nach Anspruch 1 oder 2, **dadurch gekennzeichnet, dass** der zweite Gehäuseteil (8) auf seiner dem Körper des Benutzers zugewandten Seite (22) wenigstens bereichsweise in einer horizontalen und in einer vertikalen Ebene konkav ausgebildet ist.

4. Vorrichtung nach einem oder mehreren der vorstehenden Ansprüche, **dadurch gekennzeichnet, dass** der zweite Gehäuseteil (8) auf seiner dem Körper des Benutzers zugewandten Seite in einem von einer Standseite (28) der Vorrichtung abgewandten Bereich eine Abschrägung (24) weg vom Körper des Benutzers in Richtung auf den ersten Gehäuseteil (4) aufweist und/oder der zweite Gehäuseteil (8) auf seiner dem Körper des Benutzers zugewandten Seite im Übergang zu Seitenwandungen (26) des zweiten Gehäuseteils (8) oder zu Seitenwandungen des ersten Gehäuseteils (4) abgeschrägt ausgebildet ist.

5. Vorrichtung nach einem oder mehreren der vorstehenden Ansprüche, **dadurch gekennzeichnet, dass** der erste und der zweite Gehäuseteil (4, 8) an ihren einander zugewandten Seiten Zentriermittel aufweisen.

6. Vorrichtung nach einem oder mehreren der vorstehenden Ansprüche, **dadurch gekennzeichnet, dass** die Gehäuseteile (4, 8) im wesentlichen quer zu der Trennebene (14) gegeneinander fügbar sind.

7. Vorrichtung nach Anspruch 6, **dadurch gekennzeichnet, dass** der eine Gehäuseteil (8) von oben leicht schräg zur Vertikalen auf ein Auflager des anderen Gehäuseteils (4) aufsetzbar und dann im wesentlichen quer zu der Trennebene (14) in Anlage gegen den anderen Gehäuseteil (4) schwenkbar ist.

8. Vorrichtung nach einem oder mehreren der vorstehenden Ansprüche, **dadurch gekennzeichnet, dass** das Verriegelungs- oder Hintergriffsmittel (34) von der Trennebene (14) eines der Gehäuseteile vorsteht und beim Fügen der Gehäuseteile quer zur Fügerichtung der Gehäuseteile auslenkbar und in eine Hintergriffsstellung bringbar ist.

9. Vorrichtung nach einem oder mehreren der vorstehenden Ansprüche, **dadurch gekennzeichnet, dass** das Verriegelungs- oder Hintergriffsmittel (34) durch ein manuell bedienbares Betätigungsorgan (32) in Freigaberichtung bringbar ist.

10. Vorrichtung nach Anspruch 9, **dadurch gekennzeichnet, dass** das manuell bedienbare Betätigungsorgan (32) an einer Oberseite der Vorrichtung vorgesehen und insbesondere als eindrückbarer Taster ausgebildet ist.

11. Vorrichtung nach einem oder mehreren der vorstehenden Ansprüche, **dadurch gekennzeichnet, dass** der erste und/oder zweite Gehäuseteil (4, 8) für einen manuellen Eingriff ausgebildet ist, so dass die Vorrichtung (2) oder der erste oder zweite Gehäuseteil (4, 8) hierdurch ergriffen und insbesondere der eine Gehäuseteil von dem anderen Gehäuseteil abgenommen werden kann.

12. Vorrichtung nach einem oder mehreren der vorstehenden Ansprüche, **dadurch gekennzeichnet, dass** die Standfläche der Vorrichtung von dem ersten Gehäuseteil (4) gebildet ist.

13. Vorrichtung nach einem oder mehreren der vorstehenden Ansprüche, **dadurch gekennzeichnet, dass** der erste Gehäuseteil (4) den zweiten Gehäuseteil (8) in Blickrichtung auf eine körperabgewandte Sichtseite (38) des ersten Gehäuseteils (4) zu wenigstens 90 % der Aufsichtsfläche in dieser Blickrichtung und insbesondere vollständig verdeckt.

14. Vorrichtung nach einem oder mehreren der vorstehenden Ansprüche, **dadurch gekennzeichnet, dass** an einer körperabgewandten Sichtseite (38) des ersten Gehäuseteils Bedienelemente und Anzeigeelemente (42) für die Unterdruck erzeugende Einrichtung vorgesehen sind und die Bedienelemente insbesondere in Form eines Touchscreens ausgebildet sind.

15. Vorrichtung nach einem oder mehreren der vorstehenden Ansprüche, **dadurch gekennzeichnet, dass** ein Neigungssensor bei dem ersten Gehäuseteil (4) vorgesehen ist, der über eine Steuerungseinrichtung bei einer vorgegebenen Neigung des ersten Gehäuseteils (4) zur Vertikalen veranlasst, dass die Anzeige auf einem Anzeigeelement (42) um 180° gedreht wird, so dass sie von oben durch den Benutzer ablesbar ist.

16. Vorrichtung nach einem oder mehreren der vorstehenden Ansprüche, **dadurch gekennzeichnet, dass** im wesentlichen die gesamte körperabgewandte Sichtseite (38) des ersten Gehäuseteils (4) von einer flächenhaften Abdeckung gebildet oder überfangen ist, so dass schmutzaufnehmende Fugen im Bereich der Bedienelemente vermieden werden.

## Claims

1. Device (2) for carrying on the body of a user to generate a vacuum for medical applications, in particular, for the vacuum therapy of wounds on the human or animal body, with a vacuum-producing device and a vessel (10) that is disposable after use for receiving body fluids, in particular, wound exudates suctioned out of the wound, and with a connection (12) for a suction tube leading to the body for this purpose, wherein the vacuum-producing device is disposed in or on a first housing part (4) of the device and the vessel (10) forms a second housing part (8) of the device, and the housing parts (4, 8) are separably fixed one against the other, and wherein the device (2) has fastening means so that it can be worn and carried on the body of the user wherein both housing parts (4, 8) are essentially disk-shaped and lie one against the other in an essentially vertically oriented separation plane (14), **characterized in that** the first housing part (4) is provided facing away from the body when the device is carried mobile on the body of the user and the second housing part (8) is facing the body and that on joining the first and second housing parts (4, 8), vacuum communication is also established between the vessel (10) and a vacuum-producing facility and that the housing parts (4, 8) can be separably fixed against one another by snap-in, latching, or other positive-action locking or back-gripping means (34).

2. Device according to the claim 1, **characterized in that** the second housing part (8) is formed concave toward the body of the user in at least one preferably horizontal plane on its side (22) facing the body of the user.

3. Device according to any one of the claims 1 or 2, **characterized in that** the second housing part (8) is formed concave toward the body of the user, at least in regions, in a horizontal plane and in a vertical plane on its side (22) facing the body of the user.

4. Device according to one or more of the previous claims, **characterized in that** the second housing part (8), on its side facing the body of the user in a region facing away from the standing side (28) of the device, has a bevel (24) away from the body of the user toward the first housing part (4) and/or that the second housing part (8) is formed with a bevel on its side facing the body in the transition to the side walls (26) of the second housing part (8) or to the walls of the first housing part (4).

5. Device according to one or more of the previous claims, **characterized in that** the first and the second housing parts (4, 8) have centering means on their sides facing each other.

6. Device according to one or more of the previous claims, **characterized in that** the housing parts (4, 8) can be joined one against the other essentially transversely with respect to the separation plane (14).

7. Device according to the claim 6, **characterized in that** the one housing part (8) can be placed on a contact surface of the other housing part (4) with a slightly upward inclination with respect to the vertical and can then be pivoted into contact with the other housing part (4) essentially transversely with respect to the separation plane (14).

8. Device according to one or more previous claims, **characterized in that** the locking or back-gripping means (34) project from the separation plane (14) of one of the housing parts and can be deflected transversely with respect to the joining direction of the housing parts during joining of the housing parts and can be put into a back-gripping position.

9. Device according to one or more previous claims, **characterized in that** the locking or back-gripping means (34) can be moved in a release direction by a manually operable operating element (32).

10. Device according to the claim 9, **characterized in that** the manually operable operating element (32) is provided on the top side of the device and, in particular, as a button that can be pressed in.

11. Device according to one or more of the previous claims, **characterized in that** the first and/or second housing part (4, 8) is constituted for manual gripping so that the device (2) or the first or second housing part (4, 8) can be gripped in this manner and, in particular, can be separated from the other housing part.

12. Device according to one or more of the previous claims, **characterized in that** the standing surface of the device is constituted by the first housing part (4).

13. Device according to one or more of the previous claims, **characterized in that** the first housing part (4) covers the second housing part (8) when viewed onto a visible side (38) of the first housing part (4) facing away from the body, concealing at least 90% of the visible surface in this viewing direction and, in particular, concealing it completely.

14. Device according to one or more of the previous claims, **characterized in that** operating elements and display elements (42) for the vacuum-producing facility are provided on a visible side (38) of the first housing part facing away from the body and the operating elements are constituted by a touchscreen.

15. Device according to one or more of the previous claims, **characterized in that** an inclination sensor is provided on the first housing part (4) that causes the display on a display element (42) to rotate through 180° at a defined inclination of the first housing part (4) with respect to the vertical by means of a control facility so that it can be read by the user from above.

16. Device according to one or more of the previous claims, **characterized in that** essentially the entire visible side (38) of the first housing part (4) facing away from the body is constituted or overlapped by a large-surface cover so that dirt-trapping joints in the region of the operating elements are avoided.

## Revendications

1. Dispositif (2) pouvant être porté sur le corps d'un utilisateur et servant à produire une pression négative pour des applications médicales, en particulier pour le traitement de plaies par pression négative sur le corps d'un être humain ou d'un animal, ledit dispositif étant pourvu d'un appareil produisant une pression négative et d'un récipient à usage unique (10) jetable après utilisation, destiné à contenir des liquides corporels, en particulier des sécrétions de plaie évacuées d'une plaie par aspiration, et d'un raccord (12) pour une conduite d'aspiration menant à cet effet jusqu'au corps, l'appareil produisant la pression négative étant disposé dans ou sur une première partie de boîtier (4) du dispositif et le récipient (10) constituant une deuxième partie de boîtier (8) du dispositif, et les parties de boîtier (4, 8) pouvant être fixées de manière amovible l'une contre l'autre, et le dispositif (2) présentant des moyens de fixation, de sorte que ledit dispositif peut être porté sur le corps de l'utilisateur et emporté avec ce dernier, les deux parties de boîtier (4, 8) se présentent sensiblement sous forme de disque et étant adjacentes l'une à l'autre par l'intermédiaire d'un plan de séparation (14) orienté sensiblement verticalement, **caractérisé en ce que** lorsque le dispositif est mobile sur le corps de l'utilisateur qui l'emporte avec lui, la première partie de boîtier (4) est opposée au corps, et la deuxième partie de boîtier (8) est tournée vers le corps, et lorsque la première et la deuxième partie de boîtier (4, 8) sont assemblées, une communication de pression négative est établie simultanément entre le récipient (10) et l'appareil produisant une pression négative, et **en ce que** les parties de boîtier (4, 8) peuvent être fixées de manière amovible l'une contre l'autre à l'aide de moyens de verrouillage ou de blocage arrière (34) agissant par enclenchement, par encliquetage ou autrement par coopération de formes.

2. Dispositif selon la revendication 1, **caractérisé en ce que** la deuxième partie de boîtier (8), sur sa face (22) tournée vers le corps de l'utilisateur, est concave en direction du corps de l'utilisateur dans au moins un plan de préférence horizontal.

3. Dispositif selon la revendication 1 ou 2, **caractérisé en ce que** la deuxième partie de boîtier (8), sur sa face (22) tournée vers le corps de l'utilisateur, est concave au moins par endroits dans un plan horizontal et dans un plan vertical.

4. Dispositif selon l'une ou plusieurs des revendications précédentes, **caractérisé en ce que** la deuxième partie de boîtier (8) présente sur sa face tournée vers le corps de l'utilisateur, dans une zone opposée à une face d'appui (28) du dispositif, un chanfrein (24) éloigné du corps de l'utilisateur en direction de la première partie de boîtier (4) et/ou la deuxième partie de boîtier (8), sur sa face tournée vers le corps de l'utilisateur, dans la transition vers les parois latérales (26) de la deuxième partie de boîtier (8) ou vers les parois latérales de la première partie de boîtier (4), est chanfreinée.

5. Dispositif selon l'une ou plusieurs des revendications précédentes, **caractérisé en ce que** la première et la deuxième partie de boîtier (4, 8) comprennent des moyens de centrage sur leurs faces tournées les unes vers les autres.

6. Dispositif selon l'une ou plusieurs des revendications précédentes, **caractérisé en ce que** les parties de boîtier (4, 8) peuvent être assemblées l'une contre l'autre sensiblement transversalement au plan de séparation (14).

7. Dispositif selon la revendication 6, **caractérisé en ce que** l'une des parties de boîtier (8) peut être posée du dessus, de manière légèrement inclinée par rapport à la verticale, sur un support de l'autre partie de boîtier (4), puis amenée à pivoter sensiblement transversalement au plan de séparation (14) en appui contre l'autre partie de boîtier (4).

8. Dispositif selon l'une ou plusieurs des revendications précédentes, **caractérisé en ce que** le moyen de verrouillage ou de blocage arrière (34) fait saillie du plan de séparation (14) d'une des parties de boîtier et, lors de l'assemblage des parties de boîtier, peut dévier transversalement à la direction d'assemblage des parties de boîtier et peut être amené dans une position de blocage arrière.

9. Dispositif selon l'une ou plusieurs des revendications précédentes, **caractérisé en ce que** le moyen de verrouillage ou de blocage arrière (34) peut être amené dans une direction de libération par un organe d'actionnement (32) pouvant être commandé manuellement.

10. Dispositif selon la revendication 9, **caractérisé en ce que** l'organe d'actionnement (32) pouvant être commandé manuellement est installé sur une face supérieure du dispositif et est réalisé en particulier sous la forme d'un bouton-poussoir pouvant être pressé.

11. Dispositif selon l'une ou plusieurs des revendications précédentes, **caractérisé en ce que** la première partie et/ou la deuxième partie de boîtier (4, 8) sont conçues pour une intervention manuelle, de sorte que le dispositif (2) ou la première ou la deuxième partie de boîtier (4, 8) sont ainsi saisies et en particulier l'une des parties de boîtier peut être retirée de l'autre partie de boîtier.

12. Dispositif selon l'une ou plusieurs des revendications précédentes, **caractérisé en ce que** la surface d'appui du dispositif est formée par la première partie de boîtier (4).

13. Dispositif selon l'une ou plusieurs des revendications précédentes, **caractérisé en ce que** la première partie de boîtier (4) recouvre la deuxième partie de boîtier (8) dans la direction d'observation sur une face visible (38), opposée au corps, de la première partie de boîtier (4) jusqu'à au moins 90 % de la surface exposée dans cette direction d'observation, et la recouvre en particulier intégralement.

14. Dispositif selon l'une ou plusieurs des revendications précédentes, **caractérisé en ce que** des éléments de commande et des éléments d'affichage (42) pour l'appareil produisant la pression négative sont installés sur une face visible (38), opposée au corps, de la première partie de boîtier, et les éléments de commande sont réalisés en particulier sous la forme d'un écran tactile.

15. Dispositif selon l'une ou plusieurs des revendications précédentes, **caractérisé en ce qu'**un détecteur d'inclinaison est prévu pour la première partie de boîtier (4), lequel détecteur, dans le cas d'une inclinaison prédéfinie de la première partie de boîtier (4) par rapport à la verticale, amène l'indication sur un élément d'affichage (42) à tourner de 180° par l'intermédiaire d'un appareil de commande, de sorte que ladite indication peut être lue du dessus par l'utilisateur.

16. Dispositif selon l'une ou plusieurs des revendications précédentes, **caractérisé en ce que** sensiblement toute la face visible (38), opposée au corps, de la première partie de boîtier (4) est formée ou recouverte par un revêtement surfacique, de sorte que des joints absorbant la saleté sont évités dans la zone des éléments de commande.
